# EUROPÄISCHE PATENTANMELDUNG

(11) **EP 2 281 590 A2**
(43) Veröffentlichungstag der Anmeldung: **09.02.2011**
(21) Anmeldenummer: 10168724.2
(22) Anmeldetag: 07.07.2010
(51) Int. Cl.: A61L 31/02, A61L 31/14

(54) **Biokorrodierbares Implantat mit einer aktiven Beschichtung**

(30) Priorität: 06.08.2009 US 231685 P
(71) Anmelder: Biotronik VI Patent AG, 6341 Baar (CH)
(72) Erfinder: Borck, Alexander, 91086 Aurachtal (DE)
(74) Vertreter: Lindner-Vogt, Karin L.

(57) **Zusammenfassung**

Die Erfindung betrifft ein Implantat mit einem Grundkörper aus einem biokorrodierbaren, metallischen Implantatwerkstoff mit einer aktiven Beschichtung und/oder Kavitätenfüllung, die wenigstens eine antioxidative Substanz enthält.

## Beschreibung

Die Erfindung betrifft ein Implantat mit einem Grundkörper aus einem biokorrodierbaren, metallischen Implantatwerkstoff mit einer aktiven Beschichtung und/oder Kavitätenfüllung, die wenigstens eine antioxidative Substanz enthält.

### Hintergrund der Erfindung

Implantate haben in vielfältiger Ausführungsform Anwendung in der modernen Medizintechnik gefunden. Sie dienen beispielsweise der Unterstützung von Gefäßen, Hohlorganen und Gangsystemen (endovaskuläre Implantate), zur Befestigung und temporären Fixierung von Gewebeimplantaten und Gewebstransplantationen, aber auch zu orthopädischen Zwecken, z.B. als Nagel, Platte oder Schraube.

So hat sich zum Beispiel die Implantation von Stents als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten und einen rohrförmigen Grundkörper durch den der Blutfluss ungehindert weiterläuft. Die Umfangswandung wird in der Regel von einer gitterartigen Tragstruktur gebildet, die es erlaubt, den Stent in einem komprimierten Zustand mit kleinem Außendurchmesser bis zur zu behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Der Vorgang der Positionierung und Expansion der Stents während der Prozedur und die abschließende Lage des Stents im Gewebe nach Beendigung der Prozedur muss durch den Kardiologen überwacht werden. Dies kann durch bildgebende Verfahren, wie z.B. durch Röntgenuntersuchungen erfolgen.

Das Implantat oder der Stent besitzt einen Grundkörper aus einem Implantatwerkstoff. Ein Implantatwerkstoff ist ein nicht lebendes Material, das für eine Anwendung in der Medizin eingesetzt wird und mit biologischen Systemen in Wechselwirkung tritt. Grundvoraussetzungen für den Einsatz eines Werkstoffes als Implantatwerkstoff, der bei bestimmungsgemäßer Verwendung mit der Körperumgebung in Kontakt steht, ist dessen Körperverträglichkeit (Biokompatibilität). Unter Biokompatibilität wird die Fähigkeit eines Werkstoffes verstanden, in einer spezifischen Anwendung eine angemessene Gewebereaktion hervorzurufen. Dies beinhaltet eine Anpassung der chemischen, physikalischen, biologischen und morphologischen Oberflächeneigenschaften eines Implantates an das Empfängergewebe mit dem Ziel einer klinisch erwünschten Wechselwirkung. Die Biokompatibilität des Implantatswerkstoffs ist weiterhin abhängig vom zeitlichen Ablauf der Reaktion des Biosystems, in das implantiert wird. So treten relativ kurzfristig Reizungen und Entzündungen auf, die zu Gewebeveränderungen führen können. Biologische Systeme reagieren demnach in Abhängigkeit von den Eigenschaften des Implantatswerkstoffs in verschiedener Weise. Entsprechend der Reaktion des Biosystems können die Implantatswerkstoffe in bioaktive, bioinerte und degradierbare/resorbierbare Werkstoffe unterteilt werden.

Eine biologische Reaktion auf polymere, keramische oder metallische Implantatwerkstoffe hängt von der Konzentration, Einwirkdauer und Art der Zuführung ab. Häufig führt die Gegenwart eines Implantatwerkstoffs zu Entzündungsreaktionen, deren Auslöser mechanische Reize, chemische Stoffe aber auch Stoffwechselprodukte sein können. Der Entzündungsvorgang ist in der Regel begleitet von der Einwanderung neutrophiler Granulozyten und Monozyten durch die Gefäßwände, der Einwanderung von Lymphozyten-Effektorzellen unter Bildung spezifischer Antikörper gegen den Entzündungsreiz, der Aktivierung des Komplementsystems unter Freisetzung von Komplementfaktoren, die als Mediatoren wirken und letztendlich der Aktivierung der Blutgerinnung. Eine immunologische Reaktion ist meist eng mit der Entzündungsreaktion verbunden und kann zur Sensibilisierung und Allergiebildung führen. Bekannte metallische Allergene umfassen beispielsweise Nickel, Chrom und Kobalt, die auch in vielen chirurgischen Implantaten als Legierungskomponenten Verwendung finden. Ein wesentliches Problem der Stentimplantation in Blutgefäße ist die In-Stent Restenose aufgrund eines überschießenden neointimalen Wachstums, das durch eine starke Proliferation der arteriellen glatten Muskelzellen und eine chronische Entzündungsreaktion hervorgerufen wird.

Ein aussichtsreicher Ansatz zur Lösung des Problems liegt in der Verwendung biokorrodierbarer Metalle und deren Legierungen als Implantatwerkstoff, denn zumeist ist eine dauerhafte Stützfunktion durch den Stent nicht erforderlich; das zunächst geschädigte Körpergewebe regeneriert. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Kalzium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest <1% bekannt, die sich insbesondere zur Herstellung einer Endoprothese, zum Beispiel in Form eines selbstexpandierenden oder ballonexpandierbaren Stents, eignet. Der Einsatz von biokorrodierbaren metallischen Werkstoffen in Implantaten dürfte zu einer deutlichen Minderung von Abstoßungs- oder Entzündungsreaktionen führen. Solche biokorrodierbaren Implantate und Stents weisen häufig auch eine Beschichtung oder Kavitätenfüllung mit einem geeigneten Polymer auf.

Ein Problem beim Einsatz dieser biokorrodierbaren Implantate, die ganz oder in Teilen aus einem metallischen Werkstoff bestehen, liegt darin, dass die Abbauprodukte, die bei der Korrosion des Implantats entstehen und freigesetzt werden, oft einen merklichen Einfluss auf den lokalen pH-Wert haben und zu ungewünschten Gewebsreaktionen führen können. Zudem weisen diese biokorrodierbaren Implantate auf Grund ihrer erhöhten Korrosionsrate häufig eine für den gewünschten Verwendungszweck und Implantationsort zu kurze Implantatintegrität auf. Insbesondere beim Abbau von Mg-haltigen biokorrodierbaren Implantatwerkstoffen kann es zu einem Anstieg des pH-Wertes in der unmittelbaren Umgebung kommen. Dieser Anstieg des pH-Werts kann zu einem Phänomen führen, dass unter dem Begriff Alkalose zusammengefasst wird. Der lokale pH-Wert-Anstieg führt dabei zu einem Ungleichgewicht der Ladungsverteilung in den das Gefäß umgebenden glatten Muskelzellen, was dazu führen kann, dass es zu einer lokalen Erhöhung des Muskeltonus im Bereich des Implantats kommt. Dieser erhöhte Druck auf das Implantat kann zum vorzeitigen Verlust der Implantatintegrität führen. Ist das Implantat z.B. ein Stent, so kann es im Zuge einer solchen Vasokonstriktion im Gefäßbereich um den Stent zu einer Restenose oder einer Gefäßlumenbeeinträchtigung kommen.

Um die Risikofaktoren einer Restenose zu verhindern, wurde weiterhin eine Vielzahl an Beschichtungen für Stents entwickelt, die eine erhöhte Hämokompatibilität bieten sollen. Seit längerer Zeit werden bspw. in die Beschichtung der Stents antikoagulierende, antimikrobielle, antiinflammatorische und antiproliferative Agenzien einzeln oder in Kombination eingesetzt. Diese Substanzen sollen aus dem Beschichtungsmaterial des Stents derart freigesetzt werden, dass sie Entzündungen des umliegenden Gewebes, überschießendes Wachstum der glatten Muskelzellen oder das Verklumpen von Blut verhindern. Allerdings weisen diese beschichteten Implantate in der Regel eine geringe Haltbarkeit auf, d.h. sie können nur über einen kurzen Zeitraum gelagert werden, oder benötigen zudem besondere Lagerungsbedingungen, wie beispielsweise eine Lagerung der Produkte bei 4°C. Dies führt zu einem erhöhten Ausschuss an gefertigten Produkten und somit zu einem erhöhten wirtschaftlichen Verlust.

Ein weiteres Problem bei der Optimierung wirkstoffbeladener Stents liegt in der Einstellung der Dosierung des freizusetzenden Wirkstoffs. Hierbei ist limitierend, dass die Menge des auf die Außenseite der Stents aufzubringenden Wirkstoffs stark begrenzt ist, da die für die Aufbringung bereitstehenden Flächen am Stent sehr klein sind. Bei Stents aus biokorrodierbaren Magnesiumlegierungen kann zusätzlich das Problem auftreten, dass das durch Korrosion des Werkstoffs entstehende stark alkalische Milieu das Resorptionsverhalten des aufzunehmenden Wirkstoffs negativ beeinflusst. So werden Wirkstoffe teils als Hydrochloride eingesetzt, wenn die Löslichkeit des Wirkstoffs sonst zu gering ist. Derartige Hydrochloride werden jedoch im entstehenden stark alkalischen Milieu wieder in die schlecht löslichen deprotonierten Wirkstoffe überführt.

Der vorliegenden Erfindung liegt die Aufgabe zugrunde, eines oder mehrere der zuvor geschilderten Probleme zu lösen oder zumindest zu mindern.

Insbesondere sollen die Korrosionsbeständigkeit der biokorrodierbaren Implantate sowie die Resorption der Wirkstoffe verbessert werden, wenn sie Bestandteil einer Beschichtung und/oder Kavitätenfüllung eines Implantats aus einem biokorrodierbaren Werkstoff sind. Weiterhin soll die Lagerstabilität der beschichteten Implantate verbessert werden.

Die Aufgabe wird durch Bereitstellung eines Implantats gelöst, mit einem Grundkörper, der ganz oder in Teilen aus einem biokorrodierbaren, metallischen Werkstoff besteht, wobei der Grundkörper eine Beschichtung und/oder eine Kavitätenfüllung aufweist, die aus mindestens einer antioxidativen Substanz besteht oder mindestens eine antioxidative Substanz enthält.

Der Erfindung liegt die Erkenntnis zu Grunde, dass die Integrität von biokorrodierbaren metallischen Implantaten mit einer Beschichtung oder einer Kavitätenfüllung, die aus mindestens einer antioxidativen Substanz besteht oder mindestens eine antioxidative Substanz enthält, deutlich verbessert werden kann, was zu einer längeren Standzeit der Implantate am Implantationsort führt. Weiterhin bedingt die antioxidative Substanz auf Grund ihrer antioxidativen Wirkung eine erhöhte Lagerstabilität der hergestellten Implantate.

Im Rahmen der vorliegenden Erfindung handelt es sich bei der antioxidativen Substanz bevorzugt um Squalen.

Andere geeignete Substanzen mit einer antioxidativen Wirkung sind: α-Tocopherol (Vitamin E), Retinol (Vitamin A), BHT (Butylhydroxytoluol), BHA (Butylhydroxyanisol), Ascorbinsäure (Vitamin C), Gallate wie Propylgallat (E 310), Octylgallat (E 311), Dodecylgallat (E 312), sowie Calciumdinatriummethylendiamintetraacetat (CaNa₂EDTA), Carotinoide wie Astaxanthin (E 161j), β-Carotin (E 160a), Canthaxanthin (E 161 g), Capsanthin (E 160c), Capsorubin, Cryptoxanthin, Lutein (E 161b), Luteoxanthin, Lycopin (E 160d) und Zeaxanthin (E 161h), sowie das körpereigene Peptid Glutathion (GSH), die Proteine Transferrin, Albumin, Coeruloplasmin, Hämopexin und Haptoglobin, sowie Superoxiddismutase (SOD), Glutathionperoxidase (GPX), Katalase, Lecithin (E 322), Milchsäure (E 270), Oligomere Proanthocyanidine, mehrfach-Phosphate wie Diphosphate (E 450), Triphosphate (E 451), Polyphosphate (E 452), sowie Schwefeldioxid (E 220), Natriumsulfit (E 221), Natriumbisulfit (E 222), Natriumdisulfit (E 223), Kaliumsulfit (E 224), Calciumsulfit (E 226), Calciumhydrogensulfit (E 227), Kaliumbisulfit (E 228) und Selen.

Die genannten Substanzen können den oxidativen Einfluss von Sauerstoff aus der Luft zurückdrängen oder schon gebildete Radikale eliminieren.

Squalen, auch 2,6,10,15,19,23-Hexamethyl-2,6,10,14,18,22-tetracosahexaen, Spinacen oder Supraen genannt, gehört zu der Klasse der Isoprenoiden und wird als Muttersubstanz der Triterpene (C30) angesehen und spielt eine wesentliche Rolle bei der Biosynthese von lebenswichtigen Substanzen höherer Organismen. Diese symmetrisch gebaute, aliphatische Verbindung ist vor allem Ausgangssubstanz für die Bildung der Steroide, zu denen wichtige Verbindungen wie Sterole, Gallensäuren, Steroidhormone, Vitamine der D-Gruppe, Saponine und Herzglycoside gehören. Bei der Biosynthese von beispielsweise Cholesterin wird Squalen intermediär durch reduktive Dimerisierung von Farnesyldiphosphat erhalten, welches anschließend über eine Squalenoxid-Zwischenstufe zu Lanosterin weiterreagiert, eine Vorstufe des Cholesterins.

Ein wesentlicher Vorteil von Squalen ist seine deutlich verbesserte Körperverträglichkeit im Hinblick auf Toxizität im Vergleich zu anderen Isoprenoiden oder Isoprenoid-Derivaten sowie seine verbessert Anreicherung im Körper. So wirken beispielsweise Lycopene und Ubichinon in Konzentrationen von 10 µMol/L bereits toxisch. Dahingegen wirkt Squalen auch in Konzentrationen von 100 µMol/L nicht toxisch.

Als biokorrodierbar im Sinne der Erfindung werden Legierungen und Elemente bezeichnet, bei denen in physiologischer Umgebung ein Abbau/Umbau stattfindet, so dass der aus dem Werkstoff bestehende Teil des Implantates ganz oder zumindest überwiegend nicht mehr vorhanden ist. Biokorrodierbare metallische Werkstoffe im Sinne der Erfindung umfassen Metalle und Legierungen ausgewählt aus der Gruppe umfassend Eisen, Wolfram, Zink, Molybdän und Magnesium und insbesondere solche biokorrodierbare metallische Werkstoffe, die in wässriger Lösung zu einem alkalischen Produkt korrodieren.

Beispielsweise besteht der metallische Grundkörper aus Reineisen, einer biokorrodierbaren Eisenlegierung, einer biokorrodierbaren Wolframlegierung, einer biokorrodierbaren Zinklegierung oder einer biokorrodierbaren Molybdänlegierung. Vorzugsweise besteht der metallische Grundkörper aus Magnesium. Insbesondere ist der biokorrodierbare metallische Werkstoff eine Magnesiumlegierung.

Unter einer biokorrodierbaren Magnesiumlegierung wird ein metallisches Gefüge verstanden, dessen Hauptkomponente Magnesium ist. Hauptkomponente ist die Legierungskomponente, deren Gewichtsanteil an der Legierung am höchsten ist. Ein Anteil der Hauptkomponente beträgt vorzugsweise mehr als 50 Gew.%, insbesondere mehr als 70 Gew.%. Vorzugsweise enthält die biokorrodierbare Magnesiumlegierung Yttrium und weitere Seltenerdmetalle, da sich eine derartige Legierung aufgrund ihrer physiko-chemischen Eigenschaften und hohen Biokompatibilität, insbesondere auch seiner Abbauprodukte, auszeichnet. Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71) verstanden.

Die Magnesiumlegierung ist in ihrer Zusammensetzung so zu wählen, dass sie biokorrodierbar ist. Als biokorrodierbar im Sinne der Erfindung werden Legierungen bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl₂ 0,2 g/l, KCl 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37 °C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Es wurde nun überraschenderweise festgestellt, dass ein Implantat mit einem Grundkörper der ganz oder in Teilen aus einer biokorrodierbaren Magnesiumlegierung besteht und einer Beschichtung und/oder Kavitätenfüllung, die aus Squalen besteht oder Squalen enthält, eine verbesserte Korrosionsbeständigkeit aufweist.

Der positive Einfluss der antioxidativen Substanz Squalen lässt sich über zwei mögliche Wege erklären. Werden Implantate in den Körper gebracht, so werden sofort körpereigene Maßnahmen wie z.B. die Aktivierung von Makrophagen eingeleitet. Die Makrophagen versuchen den Fremdkörper aufzunehmen, ist dieser zu groß, setzen die Makrophagen Substanzen frei, die extrem sauer und korrodierend wirken. Squalen als körpereigene Substanz verhindert die Makrophagen-Einwanderung und -Aktivierung. Eine Beschichtung mit dieser Substanz führt dazu, dass die körpereigene Reaktion erhebliche geringer ausfällt. Der Implantatgrundkörper wird nicht oder nur vermindert diesen korrosiven Substanzen ausgesetzt. Zusätzlich führt die sehr lipophile Substanz Squalen dazu, dass Wasser nur verlangsamt an das Implantat gelangt. So wird die normale Korrosion verlangsamt.

So kann die Integrität der erfindungsgemäßen Implantaten m Vergleich zu den im Stand der Technik bekannten biokorrodierbaren metallischen Implantaten von 3 Wochen bis 2 Monaten auf 9 Wochen bis 5 Monaten verlängert werden.

Implantate im Sinne der Erfindung sind über ein chirurgisches Verfahren in den Körper eingebrachte Vorrichtungen und umfassen Befestigungselemente für Knochen, beispielsweise Schrauben, Platten oder Nägel, chirurgisches Nahtmaterial, Darmklammern, Gefäßclips, Prothesen im Bereich des Hart- und Weichgewebes und Ankerelemente für Elektroden, insbesondere von Schrittmachern oder Defibrillatoren.

Vorzugsweise ist das Implantat ein Stent. Stents herkömmlicher Bauart weisen eine filigrane Stützstruktur aus metallischen Streben auf, die zur Einbringung in den Körper zunächst in einem nicht-expandierten Zustand vorliegt und die am Ort der Applikation dann in einen expandierten Zustand aufgeweitet wird. Der Stent kann vor oder nach dem Crimpen auf einen Ballon beschichtet werden.

Die aktive Beschichtung bzw. Kavitätenfüllung kann zusätzlich zur antioxidativen Substanz mindestens eine pharmazeutisch aktive Substanz (Wirkstoff) aufweisen. Insbesondere ist diese pharmazeutisch aktive Substanz ausgewählt aus der Gruppe umfassend Antiphlogistika, vorzugsweise Dexamethason, Methylprednisolon und Diclophenac; Cytostatika, vorzugsweise Paclitaxel, Colchicin, Actinomycin D und Methotrexat; Immunsuppressiva, vorzugsweise Limus-Verbindungen, weiter bevorzugt Sirolimus (Rapamycin), Zotarolimus (Abt-578), Tacrolimus (FK-506), Everolimus, Biolimus, insbesondere Biolimus A9 und Pimecrolimus, Cyclosporin A und Mycophenolsäure; Thrombozytenaggregationshemmer, vorzugsweise Abciximab und Iloprost; Statinen, vorzugsweise Simvastatin, Mevastatin, Atorvastatin, Lovastatin, Pitavastatin, Pravastatin und Fluvastatin; Estrogenen, vorzugsweise 17b-Estradiol, Daizein und Genistein; Lipidregulatoren, vorzugsweise Fibrate; Immunsuppressiva; Vasodilatatoren, vorzugsweise Sartane; Calciumkanalblocker; Calcineurininhibitoren, vorzugsweise Tacrolimus; Antiinflammatorika, vorzugsweise Imidazole; Antiallergika; Oligonucleotiden, vorzugsweise Decoy-Oligodesoxynukleotid (dODN); Endothelbildner, vorzugsweise Fibrin; Steroiden; Proteinen/Peptiden; Proliferationshemmer; Analgetika und Antirheumatika; Endothelinrezeptor-Antagonisten, vorzugsweise Bosentan; Rho-Kinase Inhibitoren, vorzugsweise Fasudil; RGD-Peptiden und zyklische RGD (cRGD) (umfassend die Sequenz Arg-Gly-Asp); und organischen Gold- oder Platinverbindungen.

Die erfindungsgemäßen Implantate mit mindestens einer weiteren pharmazeutisch aktiven Substanz zeichnen sich durch eine erhöhte Bioverfügbarkeit des Wirkstoffs aus.

Die lipophilen Eigenschaften, die schon positive Effekte auf das Korrosionsverhalten von den erfindungsgemäßen Implantaten bewirken, haben auch hilfreiche Auswirkungen auf die Wirkstoffverteilung bzw. auf die Wirkstoffpenetration. Das Squalen zieht wie ein Film auf die Gefäßinnenseite auf. Der im Squalenfilm gelöste Wirkstoff wird dadurch länger am Implantationsort fixiert und hat länger Zeit, in das gewünschte Zielgewebe zu gelangen. Die Aufnahme in die Zellen wird verbessert und damit die Verfügbarkeit erhöht.

Eine Beschichtung im Sinne der Erfindung ist eine zumindest abschnittsweise Auftragung der Komponenten auf den Grundkörper des Stents. Vorzugsweise wird die gesamte Oberfläche des Grundkörpers des Stents von der Beschichtung bedeckt. Eine Schichtdicke liegt vorzugsweise im Bereich von 1 µm bis 100 µm, vorzugsweise 3 µm bis 15 µm. Die Beschichtung besteht aus oder enthält zumindest eine antioxidative Substanz sowie gegebenenfalls mindestens eine pharmazeutisch aktive Substanz. Die Beschichtung kann weiterhin eine die antioxidative Substanz und gegebenenfalls die pharmazeutisch aktive Substanz aufnehmende Matrix, insbesondere aus einem biokorrodierbaren Polymer, beinhalten. Alternativ können die genannten Substanzen Bestandteil einer Kavitätenfüllung sein. Die Kavität befindet sich an der Oberfläche oder im Innern des Grundkörpers, so dass im letzteren Fall die Freisetzung der Substanzen erst nach Freilegung der Kavität durch Degradation des Grundkörpers erfolgt. Die antioxidative und die pharmazeutisch aktive Substanz können räumlich voneinander getrennt, ggf. auch in verschiedenen Matrices, in der Beschichtung vorliegen.

Die Matrix im Rahmen der vorliegenden Erfindung ist vorzugsweise ein biokorrodierbares Polymer, insbesondere Polydioxanon; Polyorthoester; Polyesteramide; Polycaprolacton, Polyglycoliden; Polylactiden, vorzugsweise Poly(L-lactid), Poly(D-lactid), Poly(D,L-lactid) sowie Blends, Co-Polymere und Tri-Polymere hiervon, vorzugsweise Poly(L-lactid-co-glycolid), Poly(D-L-lactid-co-glycolid), Poly(L-lactid-co-L-lactid), Poly(L-lactid-co-trimethylencarbonat); Polysacchariden, vorzugsweise Chitosan, Levan, Hyaluronsäure, Heparin, Dextran, Chodroitinsulfat und Cellulosen; Polyhydroxyvalerat; Ethylvinylacetat; Polyethylenoxiden; Polyphosphorylcholin; Fibrin; Albumin; und/oder Polyhydroxybuttersäuren, vorzugsweise ataktische, isotaktische und/oder syndiotaktische Polyhydroxybuttersäure sowie deren Blends. Ebenso nicht oder langsam degradierende Polymere wie: Polyphoshazene wie die Polyaminophoshazene oder Poly[bis(trifluroexthoxy)phosphazen], Polyurethane, wie Pellethan, oder Polyether und Polyetherblockamide, wie Pebax, und Polyamide.

In einer bevorzugten Ausführungsform weist das biokorrodierbare metallische Implantat einen beschichteten Grundkörper auf, wobei die Beschichtung und/oder die Kavitätenfüllung aus einer antioxidativen Substanz besteht.

Dabei wird beispielsweise das Implantat nach im Stand der Technik bekannten Verfahren mit Squalen überzogen. Es hat sich überraschenderweise herausgestellt, dass bei der Beschichtung von Magnesiumlegierungen mit Squalen diese auf der Implantatoberfläche zur Eigenpolymerisation neigt. Nach dem Auftragen von Squalen und Lagerung für mehrere Stunden an der Luft unter Einfluss von Sonnenlicht konnte eine Beschichtung erhalten werden, die, anders als der reine Ölfilm, erhebliche mechanische Belastungen übersteht. Der reine Ölfilm bestehend aus Squalen bewirkt schon allein eine Erhöhung der Standzeit des Implantats, weist jedoch gegenüber mechanischer Belastung eine verringerte Stabilität auf, so dass der Film schneller abgetragen wird, wodurch der Korrosionsschutz verschlechtert wird. Bedingt durch die Eigenpolymerisation des Squalenfilms bleibt der korrosionsinhibierende Effekt bestehen.

In einer weiteren bevorzugten Ausführungsform weist das biokorrodierbare Implantat einen beschichteten Grundkörper auf, wobei die Beschichtung und/oder Kavitätenfüllung mindestens eine antioxidative Substanz in einer Konzentration von 1 bis 20 % (Massenprozent bezogen auf das Implantatgewicht) enthält, insbesondere 2 bis 10 % .

Bei dieser Ausführungsform wirkt die antioxidative Substanz auf die am Implantationsort oft anzutreffenden Radikale. Diese Radikale treten bei vielen Entzündungsherden auf und führen auf unterschiedlichem Wege zum vorzeitigen Integritätsverlust des Implantats. Das schnelle Neutralisieren der Radikalspezies führt nachweislich zu einer Erhöhung der Korrosionsstabilität. Die antioxidative Wirkung von Squalen und den anderen genannten antioxidativen Substanzen ist um ein vielfaches höher als beispielsweise für eine Implantatbeschichtung mit Hyaluronsäure, welche ebenfalls neutralisierende Eigenschaften auf Radikale besitzt.

Ein weiterer Gegenstand der vorliegenden Erfindung betrifft die Verwendung von Squalen zur Herstellung eines erfindungsgemäßen Implantats.

Die erfindungsgemäßen Implantate zeichnen sich durch das Aufbringen einer Beschichtung bestehend oder enthaltend Squalen durch eine deutlich verbesserte Lagerstabilität der hergestellten Implantate aus.

Durch die erfindungsgemäße Beschichtung können kritische Arbeitschritte in der Produktion umgangen werden. Die Zeit, bis das Implantat in die Umverpackung gelangt, ist nicht mehr zeitkritisch. Sauerstoff oder Feuchtigkeitsreste im Verpackungsblister zeigen bis zu einem Jahr einen nachteiligen Einfluss auf die Eigenschaften des Implantats.

Ein weiterer Gegenstand der vorliegenden Erfindung betrifft Squalen zur Prophylaxe oder Therapie einer Restenose oder einer Gefäßlumenbeeinträchtigung in einem Gefäßabschnitt, der mit einem Stent versorgt wurde.

Die Erfindung wird nachfolgend anhand von Ausführungsbeispielen näher erläutert.

### Ausführungsbeispiel 1 - Beschichtung eines Stents mit Squalen als Reinsubstanz

Ein Stent aus der biokorrodierbaren Magnesiumlegierung WE43 (4 Gew.% Yttrium, 3 Gew.% Seltenerdmetalle außer Yttrium, Rest Magnesium und herstellungsbedingte Verunreinigungen) wird wie folgt beschichtet:

Der Stent wird von Staub und Rückständen gereinigt und in eine geeignete Stentbeschichtungsapparatur (DES Coater, Eigenentwicklung Fa. Biotronik) eingespannt. Mit Hilfe eines Airbrush Systems wird der sich drehende Stent unter konstanten Umgebungsbedingungen (Raumtemperatur; 42% Luftfeuchte) halbseitig mit Squalen beschichtet. Bei einem Düsenabstand von 20 mm ist ein 18 mm langer Stent nach ca. 2 min beschichtet. Nach Erreichen der beabsichtigten Schichtmasse wird der Stent 5 min bei Raumtemperatur getrocknet, ehe nach Drehen des Stents und erneutem Einspannen die unbeschichtete Seite auf dieselbe Weise beschichtet wird.

Eine Schichtmasse der aufgetragenen Beschichtung beträgt beispielsweise etwa 1-2 mg.

Der derart hergestellte Stent wird anschließend sofort gecrimpt (auf Ballon, falls gewünscht) und luftdicht verpackt und sterilisiert.

### Ausführungsbeispiel 2 - Mit polymerem Squalen beschichteter Stent

Analog Ausführungsbeispiel 1 wird ein mit Squalen beschichteter Stent hergestellt. Anschließend wird durch längeren Luftkontakt über 4 Std. des beschichteten Stents die Eigenpolymerisation von Squalen zugelassen.

Daraufhin wird der derart hergestellte Stent gecrimpt (auf Ballon, falls gewünscht) und luftdicht verpackt und sterilisiert.

### Ausführungsbeispiel 3 - Mit polymerem Squalen beschichteter Stent

Analog Ausführungsbeispiel 1 wird ein mit Squalen beschichteter Stent hergestellt. Abweichend vom Ausführungsbeispiel 1 enthält die Sprühlösung folgende Bestandteile:
10 ml Squalen
500 µl Triethanolamin
50 µl einer Vinylpyrrolidon-Lösung enthaltend 0,3 % Eosin Y

Die Bestandteile werden vor der Beschichtung gemischt und im Vorlagegefäß unter Lichtausschluss gerührt.

Der Stent wird wie in Beispiel 1 beschrieben beschichtet, dabei wird der Stent mit einer UV Röhre mit 360 nm belichtet. Mit Hilfe des Photoinitiators geschieht die Vernetzung direkt am Stent. Nach 2 Min. kann der Stent gedreht und von der anderen Seite analog beschichtet werden.

### Ausführungsbeispiel 4 - Mit polymerem Squalen beschichteter Stent

Tauchbeschichtung mit vernetztem Squalen.

Der Ansatz enthält folgende Bestandteile
100 Gewichtsanteile Squalen
5 Anteile ZnO (Zinkoxid)
2 Anteile Schwefel
1,2 Anteile CBS (N-Cyclohexyl-2-Benzothiazolsulfenamid)

Die Lösung wird gründlich gerührt.

Der Stent wird in diese Lösung getaucht, entnommen und für 20 Min. auf 140 °C getempert. Nach Ablauf dieser Zeit liegt das Squalen in vernetzter Form vor.

## Patentansprüche

1. Implantat mit einem Grundkörper, der ganz oder in Teilen aus einem biokorrodierbaren, metallischen Werkstoff besteht, wobei der Grundkörper eine Beschichtung und/oder eine Kavitätenfüllung aufweist, **dadurch gekennzeichnet, dass** die aus mindestens einer antioxidativen Substanz besteht oder mindestens eine antioxidative Substanz enthält.

2. Implantat nach Anspruch 1, **dadurch** charakterisiert, dass die mindestens eine antioxidative Substanz Squalen ist.

3. Implantat nach Anspruch 1 oder 2, wobei das Implantat ein Stent ist.

4. Implantat nach einem der vorherigen Ansprüche, bei dem der biokorrodierbare, metallische Werkstoff eine Magnesiumlegierung ist.

5. Implantat nach einem der vorhergehenden Ansprüche, **dadurch** charakterisiert, dass die mindestens eine antioxidative Substanz in eine polymere Trägermatrix eingebettet ist.

6. Implantat nach einem der vorherigen Ansprüche, wobei die antioxidative Substanz in einer Konzentration zwischen 1 bis 20 % in der Beschichtung oder Füllung einer Kavität vorliegt.

7. Implantat nach Anspruch 6, wobei die antioxidative Substanz in einer Konzentration zwischen 2 bis 10 % in der Beschichtung oder Füllung einer Kavität vorliegt.

8. Implantat nach einem der vorherigen Ansprüche, **dadurch gekennzeichnet, dass** die Beschichtung und/oder Kavitätenfüllung zusätzlich mindestens eine pharmazeutisch aktive Substanz aufweist.

9. Verwendung von Squalen zur Herstellung eines Implantats nach einem der Ansprüche 1 bis 8.

10. Squalen zur Prophylaxe oder Therapie einer Restenose oder einer Gefäßlumenbeeinträchtigung in einem Gefäßabschnitt, der mit einem Stent versorgt wurde.
